# EUROPÄISCHE PATENTSCHRIFT

(11) **EP 0 508 352 B1**
(45) Veröffentlichungstag und Bekanntmachung des Hinweises auf die Patenterteilung: **15.02.1995**
(21) Anmeldenummer: 92105933.3
(22) Anmeldetag: 06.04.1992
(51) Int. Cl.: C07D 221/22, C07D 201/02

(54) **Verfahren zur Herstellung von 2-Azabicyclo[2.2.1]hept-5-en-3-on**
Process for the preparation of 2-azabicyclo[2.2.1]hept-5-en-3-on
Procédé pour la préparation de la 2-azabicyclo[2.2.1]hept-5-en-3-one

(30) Priorität: 08.04.1991 CH 1034/91
(43) Veröffentlichungstag der Anmeldung: 14.10.1992
(73) Patentinhaber: LONZA AG, CH-3945 Gampel/Wallis (CH)
(72) Erfinder: Griffiths, Gareth, Dr., Visp (Kanton Wallis) (CH); Previdoli, Felix, Dr., Brig (Kanton Wallis) (CH)
(74) Vertreter: Weinhold, Peter, Dr.

(56) Entgegenhaltungen:
- CHEMICAL ABSTRACTS, vol. 111, no. 5, 31. Juli 1989, Columbus, Ohio, US;abstract no. 39161P, O. CAAMANO: 'An approach... the synthesis of 2-azabicylo[2.2.1]hept-5-ene-3-one' Seite 581 ;Spalte 2 ;
- JOURNAL OF ORGANIC CHEMISTRY Bd. 39, Nr. 4, 25. März 1973, Seiten 564 - 566; J.C. LAGT AND A.M. LEUSEN: 'Diels-Alder Cyclosadditions of Sulfonyl Cyanides with Cyclopentadiene. Synthesis of 2-Azabicyclo[2.2.1]hepta-2,5-dienes'
- JOURNAL OF ORGANIC CHEMISTRY Bd. 43, Nr. 12, 9. Juni 1978, Seiten 2311 - 2320; S. DULAGE AND R. VINCE: 'Synthesis of Carbocyclic Aminocleosides'
- J. CHEM SOC., PERKIN I Bd. 1977, Nr. 8, Seiten 874 - 884; J.R. MALPASS AND N.J. TWEDDLE: 'Reaction of Chlorosulphonyl isocyanate with 1,3-Dienes'
- J. ORG. CHEM. 1993, 58, 6129-6131

## Beschreibung

Die Erfindung betrifft ein Verfahren zur Herstellung von (±)-2-Azabicyclo[2.2.1]hept-5-en-3-on (I). 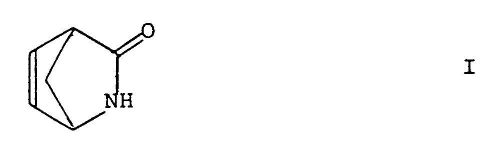
2-Azabicyclo[2.2.1]hept-5-en-3-on ist ein bicyclisches Lactam, das als Ausgangsmaterial für die Synthese von carbocyclischen Nucleosidanalogen geeignet ist (S.Daluge und R.Vince, J.Org. Chem. 43, S.2311 (1978)). Solche Nucleosidanaloge sind wegen ihrer antiviralen und chemotherapeutischen Eigenschaften als potentielle Antitumormittel von Interesse. Eine bekannte Synthese von 2-Azabicyclo[2.2.1]hept-5-en-3-on geht von 1,3-Cyclopentadien aus, das mit Chlorsulfonylisocyanat in einer 1,2-Addition ein bicyclisches N-Chlorsulfonyl-β-lactam bildet, welches sich in das entsprechende 1,4-Additionsprodukt umlagert und durch hydrolytische Abspaltung der N-Chlorsulfonyl-Gruppe in schlechter Ausbeute (27,5%) die Zielverbindung liefert (J.R.Malpass und N.J.Tweddle, J.Chem.Soc. Perkin I, 1977, S.874). Eine weitere bekannte Synthese beruht auf der Diels-Alder-Reaktion von Cyclopentadien mit p-Toluolsulfonylcyanid. Hierbei entsteht zunächst ein Tosyl-azanorbornadien, das durch saure oder alkalische Hydrolyse in die Zielverbindung übergeführt wird (J.C.Jagt und A.M.van Larsen, J.Org.Chem. 39, S.564 (1974); S.Daluge und R.Vince, loc.cit.). Nachteile dieses Verfahrens sind die Explosionsneigung von p-Toluolsulfonylcyanid und das ungünstige Mengenverhältnis des Produktes zum Nebenprodukt p-Tolylsulfinyl-p-tolylsulfon. Ausserdem wird das Cyclopentadien in grossem Ueberschuss eingesetzt, der vor der Hydrolyse abdestilliert werden muss.

Aufgabe der vorliegenden Erfindung war es, ein Verfahren zur Herstellung von 2-Azabicyclo[2.2.1]hept-5-en-3-on bereitzustellen, das von kostengünstigen Edukten ausgehend in einfacher Weise und in guter Ausbeute, ohne grosse Abfallmengen zu produzieren, das gewünschte Produkt liefert. Erfindungsgemäss wird die Aufgabe durch das Verfahren gemäss Patentanspruch 1 gelöst.

Es wurde gefunden, dass sich Cyclopentadien in stöchiometrischer Menge oder einem geringen Ueberschuss glatt mit Methansulfonylcyanid zu dem entsprechenden Diels-Alder-Addukt 3-Methansulfonyl-2-azabicyclo[2.2.1]hepta-2,5-dien (II) 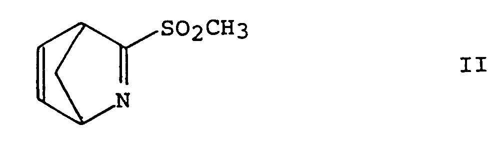
umsetzt, welches leicht zu 2-Azabicyclo[2.2.1]hept-5-en-3-on hydrolysiert werden kann. Methansulfonylcyanid kann nach bekannten Verfahren aus Methansulfonylchlorid bzw. Natriummethansulfinat hergestellt werden (M.S.A.Vrijland, Org.Synth., Coll.Vol. VI, S.727-730). Die Hydrolyse des Diels-Alder-Addukts kann sowohl unter Säure- als auch unter Basenkatalyse durchgeführt werden, vorzugsweise wird eine Säure als Katalysator eingesetzt.
Besonders bevorzugt sind Carbonsäuren, wie beispielsweise niedrige aliphatische Carbonsäuren, insbesondere Essigsäure.

Die Diels-Alder-Reaktion wird vorzugsweise in einem Lösungsmittel bei einer Temperatur von -50 bis +100°C durchgeführt. Besonders bevorzugt sind Reaktionstemperaturen von -20 bis +40°C.
Als Lösungsmittel eignen sich grundsätzlich alle Lösungsmittel, die nicht selbst mit einem der Reaktionspartner oder dem Diels-Alder-Addukt reagieren. Vorzugsweise wird ein Lösungsmittel aus der Gruppe der halogenierten aliphatischen oder aromatischen Kohlenwasserstoffe, wie beispielsweise Dichlormethan, 1,2-Dichlorethan, Chlorbenzol, der aromatischen Kohlenwasserstoffe, wie beispielsweise Benzol, Toluol, Xylol, oder der acyclischen oder cyclischen Ether, wie beispielsweise Diethylether, Diisopropylether, Methyl-tert-butylether, Tetrahydrofuran, eingesetzt. Besonders bevorzugt ist Dichlormethan.
Es ist aber auch möglich, das Cyclopentadien selbst als Lösungsmittel zu verwenden, indem es im Ueberschuss eingesetzt wird.

Das Diels-Alder-Addukt 3-Methansulfonyl-2-aza-bicyclo[2.2.1]hepta-2,5-dien kann auf übliche Weise isoliert und gegebenenfalls schonend gereinigt werden, wegen seiner geringen Stabilität wird es jedoch vorzugsweise ohne Isolierung unmittelbar der Hydrolyse unterworfen.

Die folgenden Beispiele verdeutlichen die Durchführung des erfindungsgemässen Verfahrens.

### Beispiel 1

### (±)-3-Methansulfonyl-2-azabicyclo[2.2.1]hepta-2,5-dien

Zu einer Lösung von 10,51 g Methansulfonylcyanid (100 mmol) in 30 ml Dichlormethan wurden innerhalb von 5 min bei -20°C 7,93 g Cyclopentadien (120 mmol) zudosiert. Die farblose Lösung wurde nach 2 h bei Raumtemperatur gerührt und das Lösungsmittel anschliessend abdestilliert, wobei das Diels-Alder-Addukt als gelblicher Feststoff zurückblieb.
Schmp. 52 bis 53°C
- ¹H-NMR (CDCl₃, 300 MHz): δ: 2,10 (d, J=8Hz, 1H)
2,28 (d, J=8Hz, 1H)
3,15 (s, 3H)
4,45 (m, 1H)
5,47 (m, 1H)
6,89 (m, 2H)

### Beispiel 2

### (±)-2-Azabicyclo[2.2.1]hept-5-en-3-on

Zu einer Lösung von 3,61 g Methansulfonylcyanid (97%ig, 33,3 mmol) in 30 ml Dichlormethan wurde innerhalb von 5 min eine auf -20°C abgekühlte Lösung von 2,55 g Cyclopentadien (38,6 mmol) in 10 ml Dichlormethan zudosiert. Die Reaktionstemperatur schwankte dabei zwischen 17 und 22°C. Die farblose Lösung wurde noch 2 h bei Raumtemperatur gerührt und anschliessend mit 6 ml Essigsäure versetzt. Innerhalb 1 min wurden 60 ml Wasser zugegeben und das Gemisch mit 30%iger Natronlauge neutralisiert (pH ≃ 8). Die Phasen wurden getrennt und die wässrige Phase dreimal mit je 25 ml Dichlormethan extrahiert. Die vereinigten organischen Phasen wurden über MgSO₄ getrocknet, filtriert und zur Trockene eingeengt. Der farblose feste Rückstand wurde bei 30°C/300 mbar getrocknet.
- Ausbeute:: 2,54 g, entsprechend 70% der Theorie, bezogen auf Methansulfonylcyanid.
- Reinheit (GC):: ca. 100%
Schmp. 50 bis 53°C.

## Patentansprüche

1. Verfahren zur Herstellung von 2-Azabicyclo[2.2.1]hept-5-en-3-on, dadurch gekennzeichnet, dass 1,3-Cyclopentadien mit Methansulfonylcyanid in einer Diels-Alder-Reaktion zu 3-Methansulfonyl-2-aza-bicyclo[2.2.1]hepta-2,5-dien umgesetzt und anschliessend zu 2-Azabicyclo[2.2.1]hept-5-en-3-on hydrolysiert wird.

2. Verfahren nach Patentanspruch 1, dadurch gekennzeichnet, dass die Hydrolyse in Gegenwart einer Säure durchgeführt wird.

3. Verfahren nach Patentanspruch 2, dadurch gekennzeichnet, dass als Säure eine Carbonsäure eingesetzt wird.

4. Verfahren nach Patentanspruch 3, dadurch gekennzeichnet, dass als Carbonsäure Essigsäure eingesetzt wird.

5. Verfahren nach einem oder mehreren der Patentansprüche 1 bis 4, dadurch gekennzeichnet, dass die Diels-Alder-Reaktion in einem Lösungsmittel bei einer Temperatur von -50 bis +100°C durchgeführt wird.

6. Verfahren nach Patentanspruch 5, dadurch gekennzeichnet, dass die Diels-Alder-Reaktion bei einer Temperatur von -20 bis +40°C durchgeführt wird.

7. Verfahren nach Patentanspruch 5 oder 6, dadurch gekennzeichnet, dass als Lösungsmittel ein Lösungsmittel aus der Gruppe der halogenierten aliphatischen oder aromatischen Kohlenwasserstoffe, der aromatischen Kohlenwasserstoffe oder der acyclischen oder cyclischen Ether eingesetzt wird.

8. Verfahren nach Patentanspruch 7, dadurch gekennzeichnet, dass als Lösungsmittel Dichlormethan eingesetzt wird.

9. Verfahren nach einem oder mehreren der Patentansprüche 1 bis 8, dadurch gekennzeichnet, dass das 3-Methansulfonyl-2-azabicyclo[2.2.1]hepta-2,5-dien ohne vorherige Isolierung hydrolysiert wird.

10. (±)-3-Methansulfonyl-2-azabicyclo[2.2.1]hepta-2,5-dien.

## Claims

1. Process for the preparation of 2-azabicyclo-[2.2.1]hept-5-en-3-one, characterised in that 1,3-cyclopentadiene is reacted in a Diels-Alder reaction with methanesulfonyl cyanide to give 3-methanesulfonyl-2-aza-bicyclo-[2.2.1]hepta-2,5-diene which is subsequently hydrolyzed to 2-azabicyclo-[2.2.1]hept-5-en-3-on.

2. Process according to clam 1, characterised in that hydrolysis is carried out in the presence of an acid.

3. Process according to claim 2, characterised in that a carboxylic acid is used as the acid.

4. Process according to claim 3, characterised in that acetic acid is used as the carboxylic acid.

5. Process according to one or more of claims 1 to 4, characterised in that the Diels-Alder reaction is carried out in a solvent at a temperature of from -50 to + 100 °C.

6. Process according to claim 5, characterised in that the Diels-Alder reaction is carried out at a temperature of from -20 to +40 °C.

7. Process according to claim 5 or 6, characterised in that a solvent from the group consisting of halogenated aliphatic or aromatic hydrocarbons, aromatic hydrocarbons, or acyclic or cyclic ethers is used as the solvent.

8. Process according to claim 7, characterised in that dichloromethane is used as the solvent.

9. Process according to one or more of claims 1 to 8, characterised in that 3-methanesulfonyl-2-aza-bicyclo-[2.2.1]hepta-2,5-diene is hydrolysed without being previously isolated.

10. (±)-3-Methanesulfonyl-2-aza-bicyclo-[2.2.1]hepta-2,5-diene.

## Revendications

1. Procédé pour la préparation de 2-azabicyclo [2 2.1]hept-5-ène-3-one, caractérisé en ce que du 1,3-cyclopentadiène est mis à réagir avec du cyanure de méthanesulfonyle au cours d'une réaction Diels-Alder en fournissant du 3-méthanesulfonyl-2-azabicyclo [2.2.1] hepta-2,5-diène et est ensuite hydrolysé en 2-azabicyclo[2.2.1]hept-5-ène-3-one.

2. Procédé selon la revendication 1, caractérisé en ce que l'hydrolyse s'effectue en présence d'un acide.

3. Procédé selon la revendication 2, caractérisé en ce qu'un acide carboxylique est mis en euvre en tant qu'acide.

4. Procédé selon la revendication 3, caractérisé en ce que de l'acide acétique est mis en oeuvre en tant qu'acide carboxylique.

5. Procédé selon une ou plusieurs des revendications 1 à 4, caractérisé en ce que la réaction Diels-Alder est conduite dans un solvant à une température de -50 à +100°C.

6. Procédé selon la revendication 5, caractérisé en ce que la réaction Diels-Alder s'effectue à une température de -20 à +40°C.

7. Procédé selon la revendication 5 ou 6, caractérisé en ce qu'on met en oeuvre en tant que solvant un solvant du groupe des hydrocarbures aliphatioues ou aromatiques halogénés, des hydrocarbures aromatiques ou des éthers acycliques ou cycliques.

8. Procédé selon la revendication 7, caractérisé en ce que du dichlorométhane est mis en oeuvre en tant que solvant.

9. Procédé selon une ou plusieurs des revendications 1 à 8, caractérisé en ce que le 3-méthanesulfonyl-2-azabicyclo[2.2.1]hepta-2,5-diène est hydrolysé sans isolement préalable.

10. (±)-3-méthanesulfonyl-2-azabicyclo [2.2.1]hepta-2,5-diène.
